# EUROPEAN PATENT APPLICATION

(11) **EP 0 945 519 A2**
(43) Date of publication of application: **29.09.1999**
(21) Application number: 99104051.0
(22) Date of filing: 17.03.1999
(51) Int. Cl.: C12Q 1/68

(54) **Methods for detecting differentially expressed genes**

(30) Priority: 27.03.1998 US 49569
(71) Applicant: Academia Sinica, Nan-Kang, Taipei, 115 (TW)
(72) Inventor: Peck, Konen, Taipei, 115 Taiwan (TW); Chen, Jeremy J. W., Taipei (TW); Yang, Pan-Chyr, Taipei (TW); Wu, Reen, Davis, CA 95616 (US); Chang, Fu, Hsin Chu, Taiwan (TW); Chu, Yi-Wen, Hsi Chih, Taiwan (TW); Wu, Cheng-Wen, Taipei 115, Taiwan (TW)
(74) Representative: HOFFMANN - EITLE

(57) **Abstract**

Disclosed is a method for detecting differentially expressed genes, which comprises the steps of:
(a) providing a first sample of nucleic acids representing a first population of RNA transcripts and a second sample of nucleic acids representing a second population of RNA transcripts;
(b) labeling the nucleic acids in the first sample with a first labeling element, and labeling the nucleic acids in the second sample with a second labeling element;
(c) hybridizing the labeled nucleic acids in each sample to an excess of copies of a gene-specific sequence from a DNA library;
(d) subjecting the hybridized nucleic acids in the first sample to a first enzymatic reaction and subjecting the hybridized nucleic acids in the second sample to a second enzymatic reaction, thereby generating a first chromogen from the first sample and generating a second chromogen from the second sample; and
(e) determining the amounts of the first chromogen and the second chromogen relative to each other; wherein a difference in the amounts of the first chromogen and the second chromogen indicates that the gene-specific sequence is differentially expressed in the first population of RNA transcripts and the second population of RNA transcripts.

## Description

### FIELD OF THE INVENTION

The invention relates to enzymatic reaction-based methods for simultaneously detecting differentially expressed genes.

### BACKGROUND OF THE INVENTION

The best way to find out how cellular genes act in concert to regulate cell function or transformation is to monitor the genes' fluctuating activities in normal/diseased cells and in different stages of development. Nevertheless, with the current technologies available it has only been possible to monitor the activity of one gene at a time. Monitoring the activities of a panel of genes to determine the role of genes in regulating the whole organism has always been an impregnable task. Hara *et al*., Anal. Biochem., 214, 58 (1993); Herfort *et al*., BioTechniques, 11, 598 (1991); Rhyner *et al*., J. Neurosci. Res. 16, 167 (1986); Sargent, Methods Enzymol., 152, 423 (1987); and Wieland *et al.,* Proc. Natl. Acad. Sci. USA, 87, 2720 (1990) have proposed several gene isolation methods in this respect. However, they are either slow or laborious. Up to now only a limited number of possible disease-associated genes have been identified by the traditional methods. Gene hunting has always been difficult, if not as formidable as looking for a needle in a haystack.

A gene isolation method termed "differential display" has been developed to display cDNA molecules of two different cell populations on denaturing polyacrylamide gels (Liang *et al.*, science, 257, 967 (1992)). The method is a breakthrough in gene isolation technology and is an easier and quicker method than the traditional gene isolation methods mentioned above. However, the differential display method suffers serious drawbacks such as giving frequent false positive results, being unable to identify cDNA fragments larger than 500 bases on polyacrylamide gels and to provide quantitative information of gene expression.

The Serial Analysis of Gene Expression (SAGE) (Velculescu *et al.*, Science 270, 484 (1995)) and cDNA microarray approach (Schena *et al,* Science 270, 467-470 (1995)) allow researchers to assess the activity pattern of thousands of genes simultaneously, generating in a matter of weeks information that might otherwise have taken years to gather.

The SAGE approach relies on concatenating short diagnostic sequence tags isolated from cells. The concatenated sequence tags are then cloned and sequenced. The amount of a particular sequence tag in a cell is the measure of that particular gene's activity. However, it requires large-scale sequencing efforts which may not be suitable to many molecular biology labs with limited manpower or resources.

The cDNA microarray approach requires no sequencing efforts It utilizes an arraying machine to spot expressed sequence tags (EST) or double-stranded cDNA targets on poly-L-lysine coated microscope slides. To determine expression levels of the target genes, mRNA molecules extracted from cells are labeled with fluorescent tags during reverse transcription. The labeled cDNA molecules are used as probes to hybridize to the target cDNA molecules on microscope slides.

To obtain quantitative data of gene expression, an apparatus consisting of an argon ion laser as the excitation source and a computer controlled translation stage scans the microscope slides. Schena *et al.* (1995), used laser induced fluorescence detection to quantify the expression levels of genes. Therefore, using transparent substance such as microscope slides as supporting substrate is necessary to reduce the background scattering and auto-fluorescence. Although the system is very powerful, the cost of the laser and the detection electronics for the measurement of fluorescence make the process affordable only to some research centers.

The principles of enzyme immunoassay are well established and the technique is widely used to detect proteins or antigen-antibody complexes in Western blots, ELISA, or dot blots. In the past few years, these methods have been developed to detect Southern blots or Northern blots on membrane filters by chemiluminescence using immunochemical principles to label probes (Bronstein *et al.,* Biotechnique, 8, 310-314 (1990)). Although the chemiluminescence method is very sensitive for detecting proteins or nucleic acids on membrane, it requires X-ray film to reveal the results. So far, there has been no easy multi-color detection method by chemiluminescence and the technique suffers the same nonlinear response problem in quantitation as the autoradiography method using X-ray films does.

Color forming enzymes such as alkaline phosphatase, horseradish peroxidase, and β-galactosidase are traditionally used in protein detection. Two-color detection method of two different antigens using alkaline phosphatase and horseradish peroxidase has been an established technique for many years (Lee *et al.*, Anal. Biochem., 175, 30-34 (1988)). However, the two-color detection method either involves successive probing procedures to probe one antigen at a time or requires a lengthy operation.

In DNA-related studies, multi-color detection by laser induced fluorescence has attracted much attention since the invention of the automated DNA sequencer (Smith *et al.*, Mature, 321, 674-679 (1986)). The multi-color signals in laser induced fluorescence detection are pseudo-color encoded to represent intensities. The signals must be obtained by electronic detection devices and the technique offers no way to obtain true color signals since the laser excitation is not a white light source.

There remains a need to develop methods by which it is possible to simultaneously identify multiple differentially expressed genes among thousands of genes, and are accessible to laboratories without expansive laser induced fluorescence detection devices.

### SUMMARY OF THE INVENTION

It is therefore an object of the invention to provide an enzymatic reaction-based method to simultaneously detect multiple differentially expressed genes among thousands of genes.

It is another object of the invention to provide an enzymatic reaction-based method to quantify the expression levels of genes.

### BRIEF DESCRIPTION OF THE DRAWINGS

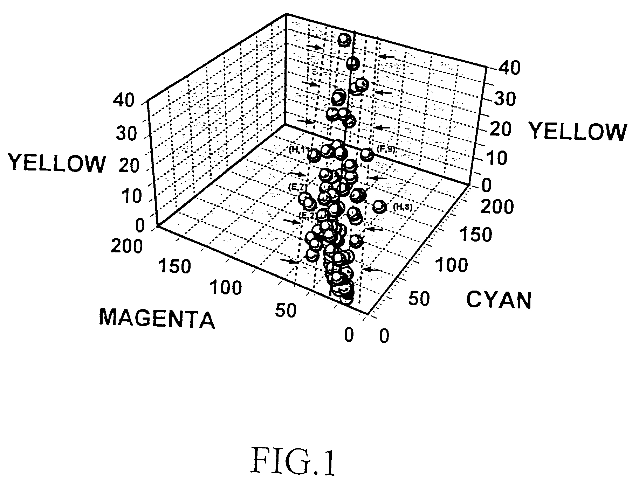

Fig. 1 is a scatter plot of the spots on a membrane produced from mixed chromogens of different colors in a method of the invention.

### DETAILED DESCRIPTION OF THE INVENTION

This invention provides methods which detect and quantify gene fragments colored by the enzymatic reaction of color forming enzymes. The true color signals generated by the color forming enzymes contain more information than just intensity. Based on the way human eyes perceive color, a color can be described by three components, hue, saturation and brightness. A slight change in any of the three components results m different colors. Like an artist mixing colors on a palette, many different colors can be generated just by mixing different proportion of two colors. The color of each gene spot on the filter membrane reflects the relative proportion of the gene probe in two different cell populations. By utilizing true color signals, this invention teaches a way not only to quantify the expression levels of genes but also to identify differentially expressed genes among thousands of genes.

Accordingly, in the first aspect, the invention relates a method for detecting differentially expressed genes, which comprises the steps of:
(a) providing a first sample of nucleic acids representing a first population of RNA transcripts and a second sample of nucleic acids representing a second population of RNA transcripts;
(b) labeling the nucleic acids in the first sample with a first labeling element, and labeling the nucleic acids in the second sample with a second labeling element;
(c) hybridizing the labeled nucleic acids in each sample to an excess of copies of a gene-specific sequence from a DNA library;
(d) subjecting the hybridized nucleic acids in the first sample to a first enzymatic reaction and subjecting the hybridized nucleic acids in the second sample to a second enzymatic reaction, thereby generating a first chromogen from the first sample and generating a second chromogen from the second sample; and
(e) determining the amounts of the first chromogen and the second chromogen relative to each other; wherein a difference in the amounts of the first chromogen and the second chromogen indicates that the gene-specific sequence is differentially expressed in the first population of RNA transcripts and the second population of RNA transcripts.

Labeling elements suitable for use in the subject invention are those well known in the art. Examples of the elements include, but not limited to, e.g., biotin, fluorescein, rhodamin, tetramethylrhodamine, Texas Red, digoxigenin, dinitrophenyl, and Cascade blue (Haugland, Handbook of Fluorescent Probes and Research Chemicals, 6^{th} ed., Molecular Probes, Inc.).

The labeling elements can be used to label the nucleic acids in the samples via methods well known in the art, e.g., via end-labeling or internally labeling techniques.

The DNA library is obtainable from any available source, e.g., commercially available DNA library or constructed microorganisms or cells having the library.

In the enzymatic reaction, a first specific binding element is used to bind with the first labeling element, and a second specific binding element, the second labeling element. The first specific binding element has an activity to convert a first chromogenic substrate into the first chromogen and the second specific binding element has an activity to convert a second chromogenic substrate into the second chromogen. Accordingly, when the first chromogenic substrate and the second chromogenic substrate are contacted with the first specific binding element and the second specific binding element, the first chromogenic substrate and the second chromogenic substrate are converted into the first chromogen and the second chromogen, respectively.

The specific binding elements are each composed of a first molecule member and a second molecule member. The first molecule member has a high affinity and specificity to the labeling element. Any molecule known to have such affinity and specificity can be used as the first molecule member. For instance, it may be an antibody directed against the labeling element, or streptavidin when biotin is used as the labeling element. The second molecule member has an activity to convert a chromogenic substrate into a chromogen. Any molecule known to have such converting activity is suitable for use in the invention. Examples of such molecules include, but not limited to, e.g. Horse Radish Peroxidase (HRP), Alkaline Phosphatase (AP), and β-galactosidase (β-Gal). The first molecule member and the second molecule member are properly combined to form the specific binding element. For instance, one example of the specific binding element is composed of strepavidin as the first molecule member and β-Gal fused to streptavidin moiety as the second molecule member.

Any chromogenic substrate well known in the art of enzymatic reactions is suitable for use in the invention. Examples of the substrate include, but not limited to, e.g. X-gal, Fast Red TR/Naphthol AS-MX, BCIP/NBT, and CN/DAB.

The chromogenic substrate and the second molecule member of the specific binding element are properly chosen such that a desired chromogen is produced when the chromogenic substrate is contacted with the specific binding element. For instance, a specific binding element comprising β-Gal as the second molecule member is chosen to convert the relatively colorless chromogenic substrate X-gal into a bluish chromogen.

The chromogen produced by the methods of the subject invention is preferably detected by digitizing an image of the chromogen, but other methods of detection can also be used. For example, the production of a specific chromogen may be detected by mass spectrometry.

In one variation of the assay, the nucleic acids in the first sample and the nucleic acids in the second sample are mixed together after the nucleic acids in the first sample are labeled with the first labeling element and the nucleic acids in the second sample are labeled with the second labeling element. In this case, the first labeling element and the second labeling element are different. For example, the first labeling element is biotin and the second labeling element is digoxigenin. In addition, the color of the first chromogen is different from the color of the second chromogen.

Alternatively, the nucleic acids in the first sample and the nucleic acids in the second sample are not mixed together and the color of the first chromogen can be the same as the color of the first chromogen.

Optionally, to improve the detection limits, the enzymatic reactions of the present invention further comprise a catalyzed reporter deposition (CARD) process, a signal amplification method described by Bobrow *et. al.,* J. Immuno. Methods, 137, 103-112, (1991).

The methods of the subject invention can be carried out but not limited on a microarray format which is constructed on a membrane or a solid support. The membrane can be any available membranes well known in the art, e.g., nylon or nitrocellulose membrane, and the solid support can be any supports well known in the art, e.g., silicon chip.

Without further elaboration, it is believed that one skilled in the art can, based on the above disclosure and the examples described below, can utilize the present invention to its fullest extent. The following examples are to be construed as merely illustrative of how one skilled in the art can practice the claimed methods and are not limitative of the remainder of the disclosure in any way.

### Example 1

This example compares the useful detection range of chemiluminescent Northern blotting and chromogenic detection in a micrcarray format and illustrates how the claimed methods can be used to quantify a specific transcript copy number.

As a first step, the ability to carry out chromogenic detection on a microarray was tested. An arraying machine fitted with stainless steel pins spotted double-stranded cDNA fragments onto a positively charged nylon membrane (Boehringer Mannheim, Mannheim, Germany). The arraying machine was a personal-computer-controlled XYZ translation system (Newport Inc., Fountain Valley, CA, Model PM500) fitted with teflon-coated steel pins for sample delivery. The arraying system was capable of placing 100 µm diameter spots on nylon membranes with spots spaced 150 µm apart. Position resolution of the spots was better than 0.1 µm. With this capacity, approximately 85,000 gene transcripts can be placed on a piece of nylon membrane measuring 35 mm by 55 mm by using a 24-pin arraying tool.

To compare the dynamic range (the linear response range of output values) of chromogenic detection with chemiluminescent Northern blotting, various predetermined amounts (9.4 X 10⁶, 1.9 X 10⁷, 3.8 X 10⁷, 7.5 X 10⁷, 1.5 X 10⁸, 3 X 10⁸, 6 X 10⁸, and 1.2 X 10⁹ molecules) of poly-adenylated rbcL RNA were doped in an aliquot of mRNA extract of CL1-0 cells (a human lung adenocarcinoma cell line), and the doped samples were individually deposited on nylon membranes. The rbcL (Ribulose 1,5-bisphosphate carboxylase large subunit) gene is a plant gene isolated from tobacco. The gene was cloned into pBluescript II (SK-) vector (Stratagene, La Jolla, CA) and amplified using two PCR primers, 5' -TAGAACTAGTGGATCCCCCGGGCTG-3' (SEQ ID No: 1) and 5' -TCACTATAGGGCGAATTGGGTACCG-3' (SEQ ID NO:2), which were near the EcoR I and Xho I restriction sites flanking the gene insert in the plasmid. The PCR conditions were as following: first cycle, 94°C for 5 min, 68°C for 5 min; second to the seventeenth cycle, 94°C for 30 sec, 68°C for 5 min; eighteenth to thirty-fifth cycle, 94°C for 30 sec, 68°C for 5 min with a 15 sec increment per cycle. The PCR was carried out in a 100 µl reaction mixture containing 10 mM Tris-HCl (pH 8.8), 1.5 mM MgCl₂, 50 mM KCl, 0.1% Triton X-100, 200 µM dNTP, 0.2 µM of each primer, and 2 µl of thermostable DNA polymerase (Elongase, BRL). The PCR products in plates were placed in microtiter plates and heated at 95°C with sufficient time to condense the product to a concentration of 2 - 3 µg/µl before spotting onto a nylon membrane.

For chromogenic detection, a panel of 93 cDNA fragments and three control plant genes (rbcL, rca [RUBISCO activase precursor], and lhc I [Photosystem I light-harvesting chlorophyll a/b-binding protein]), were amplified and deposited on a piece of nylon membrane as hybridization targets. To comply with the terms commonly used in the hybridization art, "probes" refers to the free, labeled DNA molecules in the hybridization solution while "targets" refers to DNA molecules immobilized on a solid substrate, in this case, a nylon membrane. To prepare hybridization probes, selected IMAGE cDNA clones (obtained from IMAGE consortium as described in Lennon et al., Genomics 33:151-152 [1996] through its distributor, Research Genetics, Huntsville, AL) and control plant genes were individually amplified by PCR to serve as probes.

These genes or cDNA clones were deposited into 96-well microtiter plates and amplified by PCR using primers specific to the individual gene's library construct. The PCR conditions were as following: first cycle, 94°C for 5 min, 60°C for 30 sec, 72°C for 3 min; second to thiry-fifth cycle, 94°C for 30 sec, 60°C for 30 sec, 72°C for 3 min; and 72°C for 10 min. The primers for PCR amplification of the cDNA clone, were: 5'-AGGAACAGCTATGACCATGATTACGC-3' (SEQ ID NO:3) and 5'-GGTTTTCCCAGTCACGACGTTGTAA-3' (SEQ ID NO:4) for Lafmid BA vectors, 5'-TACGACTCACTATAGGGAATTTGGCC-3' (SEQ ID NO:5) and 5'-GCCAGTGCCAAGCTAAAATTAACCC-3' (SEQ ID NO:6) for pT7T3D-Pac vectors, along with the primers described above for pBluescript II (SK-).

The amplified cDNA fragments were labeled with digoxigenin-11-dUTP by random primed labeling. 1 µg of the cDNA fragments and 4 µg of random hexamer were mixed and denatured at 98°C for 10 min before chilling on an ice/salt/ethanol mixture for 3 min. The labeling reaction was performed in a 20 µl solution containing 200 mM of HEPES buffer (pH 6.6), 50 mM Tris-HCl, 5 mM MgCl₂, 2 mM DTT, 200 µg/ml of BSA, 100 µM each of dATP, dCTP, dGTP, 65 µM of dTTP, 35 µM of digoxigenin-11-dUTP, and 2 units of Klenow DNA polymerase (Boehringer Mannheim). The reaction mixture was incubated at room temperature for 1 hour and precipitated by ammonium acetate and ethanol.

For Northern blotting with chemiluminescent detection, various amounts of rbcL mRNA, mRNA of other control plant genes, or selected IMAGE cDNA fragments were each combined with 1 µg of mRNA extracted from CL1-0 cells. The mixtures were individually spotted on a piece of nylon membrane, and the mRNA was cross-linked to the nylon membrane by UV irradiation. The membrane was pre-hybridized in hybridization buffer (6 X SSPE, 5 X Denhardt's reagent, 0.2% SDS, 0.5% BM blocking reagent [Boehringer Mannheim], and 50 µg/ml salmon sperm DNA) at 68°C for 1 hour. Specific hybridization conditions will, of course, differ from one assay to another, depending on the specific nucleic acids used in the assay. Hybridization procedures and reagents used therein are further described in Sambrook et al., Molecular Cloning: A Laboratory Manual, 2nd ed., Cold Spring Harbor Laboratory Press (1989).

Multiple Northern dot-blottings were performed, each utilizing a different probe representing either rbcL, rca, or selected IMAGE cDNA fragments at a concentration of 2 ng per ml of hybridization buffer. The reaction mixtures were sealed with the membranes in hybridization bags and incubated at 68°C for 8 - 12 hours. The membranes were then washed 2 times at room temperature with 2 X SSC containing 0.1% SDS for 5 min each, followed by 3 washes with 0.1 X SSC containing 0.1% SDS at 65°C for 20 min each. The membranes were then blocked in 1 X BM blocking reagent for 30 min at room temperature. Anti-DIG-alkaline phosphatase conjugates were diluted 15,000-fold in blocking buffer (0.1 M maleic acid, 0.15 M NaCl, and 0.3% Tween 20 at pH 7.5) containing 0.5 X BM blocking reagent and incubated with the membranes at room temperature for 45 min. The membranes were washed with blocking buffer three times for 10 min each and equilibrated with chemiluminescence substrate buffer containing 0.1 M Tris-HCl (pH 9.5), 0.1 M NaCl, and 50 mM MgCl₂ at room temperature for 5 min before being placed in the developing substrate solution (ECL Gene Image System, Amersham, Buckinghamshire, UK) for 3 - 5 min, according to the manufacturer's instructions. The membranes were then removed from the developing solution and exposed to X-ray film (Hyperfilm ECL, Amersham, Buchinghamshire, UK). The image on the X-ray film was then digitized by a flatbed scanner (Scanjet 4c, Hewlett Packard).

Ten solutions each containing mRNA extract of CL1-0 cells and different amounts of rbcL molecules (6 X 10⁷, 1.2 X 10⁸, 2.4- X 10⁸, 4.8 X 10⁸, 7.2 X 10⁸, 9.6 X 10⁸, 1.2 X 10⁹, 1.44 X 10⁹, 1.68 X 10⁹, and 1.92 X 10⁹ molecules) were labeled with biotin and used as hybridization probes. The GAPDH gene was used as an internal control to normalize the minor differences among different hybridization reactions. The results clearly demonstrate that the chromogenic detection method had better dynamic range of quantitation than the chemiluminescent Northern blotting method. For example, detection by Northem analysis resulted in an exponential relationship between the number of RBCL molecules and the X-ray film density, whereas chromogenic detection resulted in a linear relationship between the number of RBCL molecules and the intensity of chromogen dots.

In order to determine which method is more accurate, known amounts of rca and rbcL genes were spiked into the cellular mRNA extract of CL1-0. The results shown in Table I demonstrate that chromogenic detection is more accurate than Northern dot-blotting.

**Table I**

| Probe Ratio | Experimental Results | | Probe Quantity |
|---|---|---|---|
| | Microarray/CD | Northern Blotting | |
| rca/rbcl = 4:1 | 3.1:1.0 | 1.6:1.0 | High quantity (1200:300) |
| rca/rbcL = 1:4 | 1.0:3.9 | 1.0:2.3 | Low quantity (120:480) |
| rca/rca = 10:1 | 6.2:1.0 | 3.7:1.0 | Large difference (1200:120) |
| rbcL/rbcL = 1:1.6 | 1.0:1.9 | 1.0:1.0 | Small difference (300:480) |

Numbers in parenthesis are the approximate number of control gene molecules spiked per cell equivalent.

The rca/rbcL results indicate that the response range of the Northern reached saturation at high gene transcript numbers such that the probe ratio of the two genes was compressed. In fact, the apparent linear relationship of probe number to chromogenic output in this range makes any interpretation of results here much easier than if Northern blotting is used instead.

The detection limit of the system was about 50 million molecules. For gene expression quantitation using 10⁶ cells, the detection limit of the system was therefore tens of transcripts per cell. On average, 25% of the cellular mRNA mass consists of transcripts expressed at less than 5 copies per cell. To detect gene transcripts at this level, 10⁷ cells may be necessary to achieve detection limit by the present protocol.

Since all that is required in this assay is that the chromogenic signals are proportional to the number of probes bound to the membrane after hybridization, a variety of labeling procedures can be used, including random-primed and end labelling. However, it is preferred that random-primed labeling be used.

### Example 2

The example illustrates how the methods of this invention can be used to identify differentially expressed genes.

Membranes containing an array of immobilized gene targets which has been hybridized to labeled probes from two mRNA sources can be prepared and used in the following manner.

Human lung adenocarcinoma cell lines CL1-0 and CL1-5 were grown in RPMI medium with 10% FBS and incubated at 37°C, 20% O₂, and 5% CO₂. These cells were used as mRNA sources and are described in Chu et al., Am J Respir Cell Mol Biol 17:353-360 (1997).

Normal human lung cDNA samples, obtained as a commercial cDNA library constructed on the Uni-ZAP XR phage vector (Stratagene, La Jolla, CA), were used to prepare the target DNA. To plate the phagemids, 200 µl of SOLR bacteria (Stratagene) (O.D. at 600 nm = 1) and an aliquot of the phage stock were mixed and incubated at 37°C for 15 min. 50 µl of the above mixture were plated on Blue/White color selection plates containing LB broth, 1.5% agarose, 50 µg/ml ampicillin, 0.5 mM IPTG (isopropyl-1-thio-β-D-galactoside), and 20 µg/ml X-gal (5-bromo-4-chloro-3-indolyl-β-D-galactoside, obtainable from Sigma as product no. B4527; see Horwitz et al., J Med Chem 7:574 [1964]). After growing at 37°C overnight, the colonies were picked from the selection plates and inoculated in 96-well microtiter plates containing 100 µl LB medium and 50 µg/ml of ampicillin. The liquid cultures were incubated with gentle shaking at 37°C overnight. 1 µl of bacteria culture from each well of the 96-well plates was deposited in one well of a "V"-bottomed 96-well polycarbonate microtiter plate and amplified by PCR using the pBluescript primers described in Example 1. The PCR conditions were as following: first cycle, 94°C for 5 min, 68°C for 5 min; second to seventeenth cycle, 94°C for 30 sec, 68°C for 5 min; eighteenth to thirty-fifth cycle, 94°C for 30 sec, 68°C for 5 min with a 15 sec increment each cycle. Other conditions for PCR and preparation of the samples for hybridization were as described in Example 1.

The three plant genes, lhc I, rbcL, and rca; and one human GAPDH (Glyceraldehydes-3-phosphate dehydrogenase) gene were amplified from tobacco cells or human adenocarcinoma cells as described in Example 1.

In order to study the interactions of known cellular genes in cells under stress or external stimulation, putative gene clones were obtained from the IMAGE consortium as described in Example 1. These gene were derived from various tissues and cloned into different library constructs. Most of the clones have been partially sequenced and the sequence information is available as expressed sequence tags (EST) from dbEST (Boguski et al., Nat Genet 4:332-333 [1993]) or GenBank (Benson et al., Nucleic Acids Res 22:3441-3444 [1994]). The cDNA clones and genes were amplified as described in Example 1.

To prepare hybridization probes, messenger RNAs were extracted from the two human lung adenocarcinoma cell lines, CL1-0 and CL1-5, by the method described in Example 1. 1 µg of CL1-0 mRNA was labeled with biotin, and 1 µg of CL1-5 mRNA was labeled with digoxigenin. The labeling reactions were done via incorporation by reverse transcription in the presence of 6 µM random primers, 0.5 mM each of dATP, dCTP, dGTP, 40 µM dTTP, 40 µM biotin-16-dUTP or 40 µM digoxigenin-11-dUTP (Boehringer Mannheim), 10 mM DTT, 0.5 unit/µl RNasin (GIBCO-BRL), and 200 units of MuMLV reverse transcriptase (GIBCO-BRL). The 50 µl reaction mixture was incubated at 40°C for 90 min and was stopped by heating the reaction mixture to 99°C for 5 min. Residual RNA was degraded by adding 5.5 µl of 3 M NaOH followed by a 30-min incubation at 50°C, and the labeled samples were neutralized by adding 5.5 µl of 3 M acetic acid. The single-stranded cDNA probes were precipitated by adding 50 µl of 7.5 M ammonium acetate, 20 µg of linear polyacrylamide as carrier, 375 µl of absolute alcohol, and water to obtain a total volume of 525 µl.

The membrane carrying the double-stranded cDNA targets was pre-hybridized in hybridization buffer (5 X SSC, 0.1% N-laurylsarcosine, 0.1% SDS, 1% BM blocking reagent, and 50 µg/ml salmon sperm DNA) at 68°C for 1 hour. The two cDNA probes were mixed in equal proportions in 10 µl hybridization buffer containing 200 µl/ml d(A)₁₀ and 300 - 400 µg/ml of human COT-1 DNA to prevent non-specific binding, and hybridized to the normal lung cDNA fragments on the membrane by Southern hybridization procedures. The labeled cDNAs from the two cancer cell lines were sealed with the membrane in a assembly (SureSeal, Hybaid, Middlesex, UK) attached to a weight, and incubated at 95°C for 2 min then at 68°C for 12 hours. The membrane was then washed with 2 X SSC containing 0.1% SDS for 5 min at room temperature followed by 3 washes with 0.1 X SSC containing 0.1% SDS at 65°C for 15 min each.

After hybridization, the membrane was blocked by 1% BM blocking reagent containing 2% dextran sulphate at room temperature for 1 hour and then rinsed with 1 X TBS buffer solution (10 mM Tris-HCI [pH 7.4] and 150 mM NaCl) containing 0.3% BSA. To detect the spots on the membrane, streptavidin/β-galactosidase enzyme conjugate and antidigoxigenin/alkaline phosphatase antibody-enzyme conjugate were employed. The detection can also be done in single-color mode. In this case, either one of the antibody/enzyme conjugates can be used. The membrane was incubated with a mixture containing 700 X diluted streptavidin-β-galactosidase (GIBCO-BRL), 10,000 X diluted anti-DIG-AP (Boehringer Mannheim), 4% polyethylene glycol 8000 (Sigma, St. Louis, MO), and 0.3% BSA in 1 X TBS buffer for 2 hours. The chromogens were generated by first treating the membrane with X-gal solution, a β-galactosidase substrate, containing 1.2 mM X-gal, 1 mM MgCl₂, 3 mM K₃Fe(CN)₆, 3 mM K₄Fe(CN)₆ in 1 X TBS buffer for 45 min at 37°C. The membrane was then briefly rinsed with deionized water and stained with Fast Red TR/Naphthol AS-MX substrate (Pierce, Rockford, USA; also obtainable from Sigma as product no. F4648), an alkaline phosphatase substrate. The color development reactions were then stopped by 1 X PBS containing 20 mM EDTA. After color development, the cDNA molecules labeled with biotin yielded a blue chromogen and the cDNA molecules labeled with digoxigenin yielded a red chiomogen. To determine the results from arrays of different densities, we performed image digitization using three different types of imaging devices that are commonly available to research laboratories: a flatbed scanner (Scanjet 4c, Hewlett Packard, Palo Alto, CA), a color video camera (JVC TK-880U, Tokyo, Japan) attached to a copy stand, and a digital camera (DCS-420, Kodak, Rochester, NY) attached to a stereomicroscope (Zeiss, Stemi 2000C, Germany). The flatbed scanner, which was the least expensive, digitized the images at 600 dots-per-inch (dpi). Although the scanner did not provide presentation quality images for spots with diameters on the order of 200 µm, it did provide sufficient image resolution for quantitation purposes. The digital camera had the highest resolution of all the devices we tested and was used to digitize spots with diameter on the order of 100 µm or less. The colors of the spots were then separated into the artists' subtractive primaries (cyan, magenta, and yellow) and quantified by computer.

Most of the spots appeared purple indicating the presence of genes commonly expressed in the two cell lines. However, some spots exhibit more distinctive colors, either redder or bluer, which was used as an indication of differentially expressed genes in the two cell lines. The gene expression pattern was reproduced in repeated experiments.

The 3-D scatter plot of Fig. 1 is a quantitative representation of the spots on the membrane described immediately above and demonstrates how the system identifies differentially expressed genes. The color of each spot on the membrane is represented in the 3-D space by the position of the ball representing the spot. As seem in Fig. 1, most of the balls cluster along the solid line, which indicates that most of the spots on the membrane have the same proportion of the three subtractive primary colors but of different intensities. Some balls lie farther away from the line than others. These outlying balls represent the spots that are composed of unequal amounts of the labeled cDNA from the two cell lines, which suggests that the target gene is differentially expressed in the two cell lines. Therefore, by closing in the threshold planes (represented by the dashed lines) in the color space towards the regression (solid) line of the sample population, the computer program can pick out and register, one by one, the spots of a particular color intensity and composition, thereby identifying particular spots as outliers. For example, spots (H, 8) and (E, 7) represent putative differentially expressed genes.

To determine optimal threshold planes such that a false positive error is less likely, an analysis of the experimental error was performed. In this context, a false positive error refers to counting a gene as differentially expressed when, in fact, it is not differentially expressed. To calibrate the standard error range of the system, cDNA molecules derived from the CL1-0 cell line were split in halves, one half labeled with biotin and the other half labeled with digoxigenin. The two halves were mixed in equal proportions and hybridized to the target cDNA molecules on the membrane. The standard error (defined as the square root of [variance/number of observations]) of the system was then estimated from this control experiment. The regression lined derived from the 96 data points is flanked by lines defining the edges of the 99% prediction interval for the systematic error. Spots lying beyond the 99% prediction interval can be observed. These spots are significant enough to be registered as candidate differentially expressed genes.

To quantify the relative expression levels of known genes in two different cell populations, the labeling procedure described above was modified to improve accuracy. For such an application, three plant genes were included in cDNA probe preparations during reverse transcription. The concept and procedures are described as follows.

In order to estimate the relative expression level of a gene in two different cell populations, the RNA extraction efficiency was taken into account. For this purpose, a known amount of lhc I poly-adenylated RNA was included in a suspension of 10⁶ cells and subjected to cell lysis, RNA extraction, reverse transcription, and labeling along with the rest of the sample. To calculate the extraction efficiency, a known amount of rbcL poly-adenylated RNA was added to the sample after the RNA extraction. The extraction efficiency could then be calculated from the chromogenic signals produced by the two plant gene probes. A known amount of biotin labeled cDNA of a third chloroplast gene, rca, was included in the probe solution before hybridization to determine the labeling efficiency. The rca gene was also used to normalize the signals on different membranes.

To obtain expression patterns in a different cell types or in the same cell type under different environments, the lhc I, rbcL, and rca plant control genes were deposited on one membrane. The expression level of a particular gene was calculated by reference to the CMY component of the control spots. As an illustration of the method and to simplify the data analysis, single color analysis was used in this experiment, the results of which are shown in Table II.

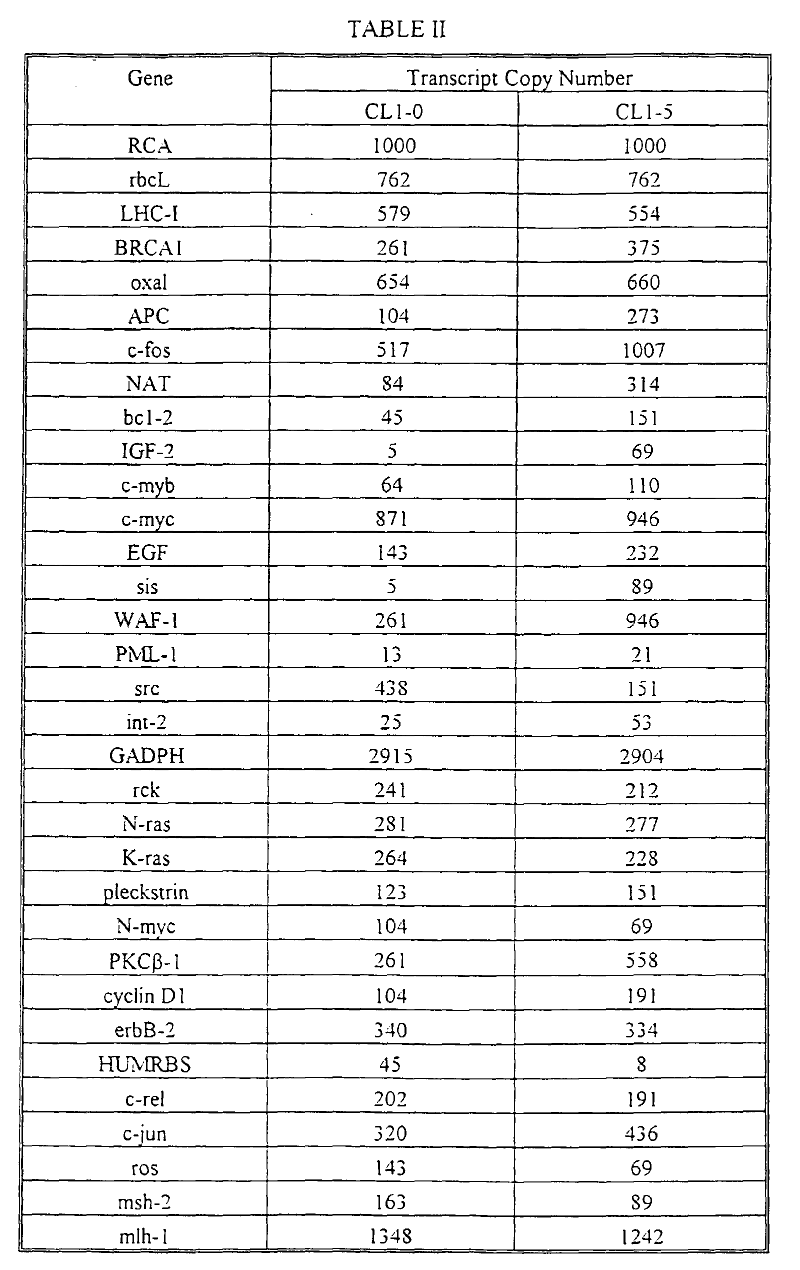

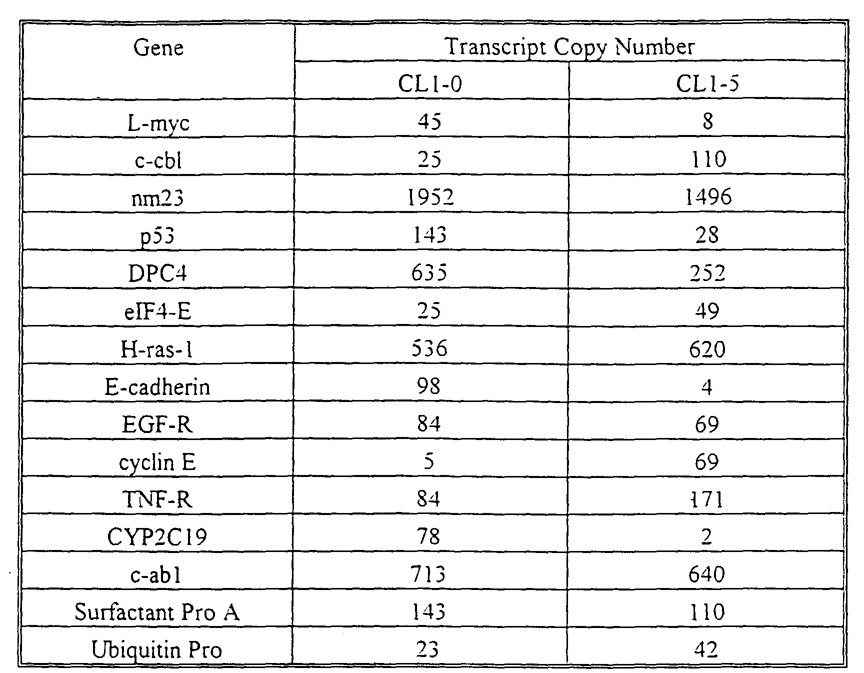

The cDNA molecules extracted from CL1-0 and CL1-5 were labeled with biotin as described above. Duplicate membranes carrying the same target gene fragments were hybridized with labeled cDNA probes derived from CL1-0 and CL1-5.

The nm23 gene is relatively over-expressed in CL1-0 cells while the PKC-β1 gene is relatively overexpressed in CL1-5 cells. These observations are consistent with previous findings which show that suppression of the nm23 gene leads to higher metastatic potential (Steeg et al., Cancer Res 48:6550-6554 [1988]) and that PKC-β1 is relatively over-expressed in the invasive cells (Schwartz et al., J Natl Cancer Inst 85:402-407 [1993]).

### Example 3

This example illustrates how the methods of this invention were used to identify genes correlated with tumor cell invasion ability.

500 genes selected from the IMAGE consortium human cDNA libraries were used as target DNA as previously described in Example 2. The probes were derived from human adenocarcinoma cell lines CL1-0, CL1-1, and CL1-5, each of increasing invasive ability, respectively. The cDNA representing the mRNA extracted from these cell lines were labeled with digoxigenin and the assay performed as in Example 2. Out of the 500 genes examined, seven genes appeared to correlate with invasiveness (Table III).

**TABLE III**

| Gene | Expression Level (Arbitrary Units) | | |
|---|---|---|---|
| | CL1-0 | CL1-1 | CL1-5 |
| sp2 TF | 6.4 | 2.7 | 0.5 |
| RAF | 17.0 | 16.0 | 3.7 |
| EDNRB | 15.5 | 13.0 | 5.0 |
| ALOX12 | 13.8 | 5.0 | 1.3 |
| nm23 | 11.9 | 10.6 | 3.1 |
| int-2 | 13.5 | 10.4 | 2.1 |
| γc precursor | 10.1 | 4.5 | 2.1 |

To determine if these same seven genes correlate with invasiveness in other mRNA samples, mouse melanoma cell lines B16F0, B16F1, and B16F10, each with increasing tumor invasion capacity, were next tested. Out of the same 500 genes examined, 15 genes (see Table IV) appeared to correlate with invasiveness.

**TABLE IV**

| Gene | Expression Level (Arbitrary Units) | | |
|---|---|---|---|
| | B16-F0 | B16-F1 | B16-F10 |
| cyclin Al | 9836 | 5374 | 3042 |
| HDLCl | 7301 | 4137 | 3964 |
| Tyr Kinase | 3133 | 703 | 461 |
| Prohibitin | 4649 | 1860 | 1481 |
| Gu4 | 2723 | 1740 | 1108 |
| RAF | 8746 | 8182 | 3949 |
| v-kit | 3407 | 1922 | 1538 |
| HSP70 | 3503 | 3057 | 2405 |
| Villin 2 | 13244 | 2448 | 1844 |
| Keratin 5 | 10,326 | 2271 | 295 |
| N-ras | 8969 | 4337 | 3058 |
| RBAPN | 6464 | 5384 | 3015 |
| eIF4-E | 4416 | 3495 | 1655 |
| H-ras-l | 7509 | 3601 | 2937 |
| nm23 | 4157 | 2487 | 1369 |

Inspecting the identity of the gene hits in Tables III and IV, only one gene, the nm23 gene, was found to correlate with invasiveness in both systems. As it is known that the nm23 gene is a metastasis suppressor gene, the result immediately above demonstrates that the microarray/CD can be used to isolate valid differentially expressed genes.

### Example 4

The example illustrates how the methods of this invention were used to identify differentially expressed genes in the same cell under different growth conditions.

To study the effect of external stimulants such as drugs or chemicals on gene expression, human trachea epithelial cells were grown with or without vitamin A acid (retinoic acid) in culture and used as mRNA sources. Table V lists genes differentially expressed when the cells were grown with or without Vitamin A acid.

**TABLE V**

| Gene | Expression Level (Arbitrary Units) | |
|---|---|---|
| | Vitamin A- | Vitamin A+ |
| MMP-1 | 16 | 141 |
| PTP-Z | 18 | 83 |
| Gro2 | 7 | 32 |
| PCNA | 6 | 26 |
| NERF-2 | 7 | 28 |
| CD71 | 15 | 57 |
| DPC4 | 23 | 81 |
| ELGF | 15 | 51 |
| AMF-R | 12 | 40 |
| EGF-R | 51 | 158 |
| NAIP | 9 | 28 |
| Ear-1 | 103 | 30 |
| L-myc | 15 | 4 |
| Ras-like protein | 132 | 18 |

It is to be understood that while the invention has been described in conjunction with the detailed description, the foregoing description is intended to illustrate and not limit the scope of the invention, which is defined by the scope of the claims. Other aspects, advantages, and modifications are within the scope of this invention.

For example, the computer program used to practice the methods of this invention can include algorithms for image processing and analysis, object recognition, color separation, and color filtering. Alternatively, patterns of gene expression can be visually inspected by the human eye. In addition, since the DNA on solid supports, such as a nylon membrane, can be preserved indefinitely, the genes of interest can be retrieved from the colored cDNA spots for further analysis such as sequencing.

## Claims

1. A method for detecting differentially expressed genes, comprising:
(a) providing a first sample of nucleic acids representing a first population of RNA transcripts and a second sample of nucleic acids representing a second population of RNA transcripts;
(b) labeling the nucleic acids in the first sample with a first labeling element, and labeling the nucleic acids in the second sample with a second labeling element;
(c) hybridizing the labeled nucleic acids in each sample to an excess of copies of a gene-specific sequence from a DNA library;
(d) subjecting the hybridized nucleic acids in the first sample to a first enzymatic reaction and subjecting the hybridized nucleic acids in the second sample to a second enzymatic reaction, thereby generating a first chromogen from the first sample and generating a second chromogen from the second sample; and
(e) determining the amounts of the first chromogen and the second chromogen relative to each other; wherein a difference in the amounts of the first chromogen and the second chromogen indicates that the gene-specific sequence is differentially expressed in the first population of RNA transcripts and the second population of RNA transcripts.

2. The method of claim 1, which is carried out in an array format with spatial density over 100 spots per cm².

3. The method of claim 2, wherein the microarray is constructed on a membrane.

4. The method of claim 3, wherein the membrane is a nylon membrane.

5. The method of claim 3, wherein the membrane is an organic polymer membrane.

6. The method of claim 1, wherein the enzymatic reactions comprise binding a first specific binding element to said first labeling element and binding a second specific binding element to the second labeling element, wherein the first specific binding element has an activity to convert a first chromogenic substrate into the first chromogen and the second specific binding element has an activity to convert a second chromogenic substrate into the second chromogen.

7. The method of claim 6, wherein the enzymatic reactions further comprise contacting the first chromogenic substrate and the second chromogenic substrate with the first specific binding element and the second specific binding element, thereby converting the first chromogenic substrate and the second chromogenic substrate into the first chromogen and the second chromogen, respectively.

8. The method of claim 1, wherein the quantity of chromogen is determined by digitizing the image of the chromogen and measuring the intensity of the chromogen image.

9. The method of any of claims 1 to 8, wherein the nucleic acids in the first sample and the nucleic acids in the second sample are mixed together after step (b) is performed and wherein the color of the first chromogen is different from the color of the second chromogen.

10. The method of claim 9, wherein the nucleic acids in the first sample and the nucleic acids in the second sample are end-labeled or internally labeled in step (b).

11. The method of claim 10, wherein the first labeling element is biotin.

12. The method of claim 10, wherein the second labeling element is digoxigenin.

13. The method of claim 9, wherein the first specific binding element is streptavidin/β-galactosidase enzyme conjugate.

14. The method of claim 9, wherein the second specific binding element is anti-digoxigenin/alkaline phosphatase antibody-enzyme conjugate.

15. The method of claim 9, wherein the first chromogenic substrate is X-gal.

16. The method of claim 9, wherein the second chromogenic substrate is Fast Red TR/Naphthol AS-MX.

17. The method of any one of claims 1 to 8, wherein the nucleic acids in the first sample and the nucleic acids in the second sample are not mixed together and wherein the color of the first chromogen is the same as the color of the second chromogen.

18. The method of claim 17, wherein both the first and the second labeling elements are biotin or digoxigenin.

19. The method of claim 17, wherein both the first and the second specific binding elements are streptavidin/β-galactosidase enzyme conjugate or anti-digoxigenin/alkaline phosphatase antibody-enzyme conjugate.

20. The method of claim 17, wherein both the first and the second chromogenic substrates are X-gal or Fast Red TR/Naphthol AS-MX.


SEQUENCE LISTING

<110> ACADEMIA SINICA

<120> Methods for detecting differentially expressed genes

<130> 77259m3

<140> 99104051.0
<141> 1999-03-17

<150> US 09/049,569
<151> 1998-03-27

<160> 6

<170> PatentIn Ver. 2.0

<210> 1
<211> 25
<212> DNA
<213> Artificial Sequence

<220>
<223> Description of Artificial Sequence: synthetic
      oligomer

<400> 1
tagaactagt ggatcccccg ggctg                                        25

<210> 2
<211> 25
<212> DNA
<213> Artificial Sequence

<220>
<223> Description of Artificial Sequence: synthetic
      oligomer

<400> 2
tcactatagg gcgaattggg taccg                                        25

<210> 3
<211> 27
<212> DNA
<213> Artificial Sequence

<220>

<223> Description of Artificial Sequence: synthetic
      oligomer

<400> 3
aggaaacagc tatgaccatg attacgc                                          27

<210> 4
<211> 25
<212> DNA
<213> Artificial Sequence

<220>
<223> Description of Artificial Sequence: synthetic
      oligomer

<400> 4
ggttttccca gtcacgacgt tgtaa                                            25

<210> 5
<211> 26
<212> DNA
<213> Artificial Sequence

<220>
<223> Description of Artificial Sequence: synthetic
      oligomer

<400> 5
tacgactcac tatagggaat ttggcc                                           26

<210> 6
<211> 25
<212> DNA
<213> Artificial Sequence

<220>
<223> Description of Artificial Sequence: synthetic
      oligomer

<400> 6
gccagtgcca agctaaaatt aaccc                                            25